(19) Europäisches Patentamt
European Patent Office
Office européen des brevets

(11) Veröffentlichungsnummer: **0 224 445 B1**

(12) **EUROPÄISCHE PATENTSCHRIFT**

(45) Veröffentlichungstag der Patentschrift: **04.09.91**

(21) Anmeldenummer: **86810532.1**

(22) Anmeldetag: **20.11.86**

(51) Int. Cl.⁵: **C09B 67/22**, C09B 57/00, C09B 63/00, C07D 487/04, C08K 5/34, //(C07D487/04, 209:00,209:00)

(54) **Pyrrolo-pyrrol Pigmentpräparate, Verfahren zu deren Herstellung sowie ihre Verwendung.**

(30) Priorität: **26.11.85 CH 5054/85**

(43) Veröffentlichungstag der Anmeldung:
**03.06.87 Patentblatt 87/23**

(45) Bekanntmachung des Hinweises auf die Patenterteilung:
**04.09.91 Patentblatt 91/36**

(84) Benannte Vertragsstaaten:
**CH DE FR GB IT LI**

(56) Entgegenhaltungen:
**EP-A- 0 014 458**
**EP-A- 0 039 307**
**EP-A- 0 061 426**
**EP-A- 0 094 911**
**EP-A- 0 133 156**
**EP-A- 0 181 290**
**EP-A- 0 184 982**

(73) Patentinhaber: **CIBA-GEIGY AG**
**Klybeckstrasse 141**
**CH-4002 Basel(CH)**

(72) Erfinder: **Jost, Max**
**Rebgartenweg 20**
**CH-4104 Oberwil(CH)**
Erfinder: **Iqbal, Abul, Dr.**
**Im Schaiengarten 1**
**CH-4107 Ettingen(CH)**
Erfinder: **Rochat, Alain Claude, Dr.**
**Route de Schiffenen 38**
**CH-1700 Fribourg(CH)**

**EP 0 224 445 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Stoffkompositionen auf der Basis von Pyrrolopyrrolen, ein Verfahren zu deren Herstellung sowie ihre Verwendung zum Färben von hochmolekularem organischem Material.

Diketo-pyrrolo-pyrrole stellen bekannte Verbindungen dar, die beispielsweise im US Patent Nr. 4'415'685 oder in der europäischen Patentanmeldung Veröffentlichungsnummer 0'133'156 als Pigmente zum Färben von organischen Polymeren beschrieben sind. Bestimmte ihrer Pigmenteigenschaften sind jedoch nicht immer befriedigend. Es wurde nun gefunden, dass mit der Einführung ausgewählter Gruppen in ihr Molekül unerwartete Verbesserungen gewisser Pigmenteigenschaften erzielt werden können.

Gegenstand der vorliegenden Erfindung sind demnach Stoffkompositionen enthaltend

a) ein Pyrrolo-pyrrol der Formel I

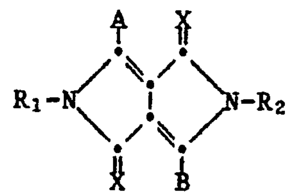

(I)

worin A und B gleiche oder voneinander verschiedene Alkyl-, Aralkyl-, Aryl- oder heterocyclische aromatische Reste, $R_1$ und $R_2$ H-Atome, unsubstituierte oder substituierte Alkylreste, ferner Alkenyl-, Alkinyl-, Aralkyl-, Cycloalkyl-, Carbamoyl-, Alkylcarbamoyl-, Arylcarbamoyl-, Alkoxycarbonyl-, Aryl-, Alkanoyl- oder Aroylgruppen und X ein O- oder S-Atom bedeuten, und

b) ein Pyrrolo-pyrrol der oben angegebenen Formel I, das mindestens eine Gruppe der Formeln $-SO_3L$, $-CO_2L$, $-PO_3(L)_2$, $-N(R_4)(R_5)$,

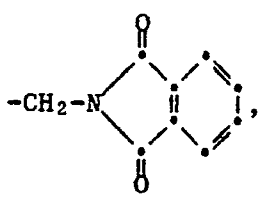, 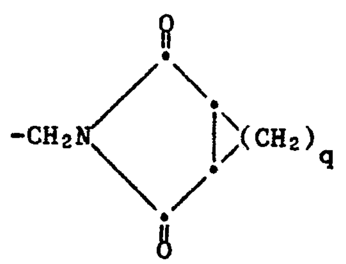, 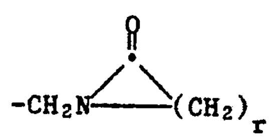 oder -NHCOR

aufweist, wobei L -H, eine Gruppe der Formeln

$$\frac{M^{+n}}{n}$$

oder $N^+H(R_3)(R_4)(R_5)$ darstellt, M ein ein-, zwei- oder dreiwertiges Metallkation, n die Zahlen 1, 2 oder 3, $R_3$, $R_4$ und $R_5$ unabhängig voneinander -H, Alkyl-, Aralkyl-, $C_5$-$C_6$-Cycloalkyl- oder Arylreste bedeuten oder $R_4$ und $R_5$ zusammen mit dem N-Atom einen 5- oder 6-gliedrigen heterocyclischen Rest bilden, q die Zahlen 0 bis 4, r die Zahlen 3 bis 5 und R Aryl oder $C_1$-$C_4$-Alkyl darstellen.

Die Reste A und B sind vorzugsweise identisch und bedeuten bevorzugt Aryl.

Bedeuten A, B, $R_1$ und $R_2$ unsubstituierte Alkylgruppen, so können diese verzweigt oder unverzweigt sein und weisen vorzugsweise 1-18, insbesondere 1-12, und besonders bevorzugt 1-6 C-Atome, auf. Als Beispiele seien Methyl, Aethyl, Isopropyl, sek.-Butyl, tert.-Butyl, tert.-Amyl, Octyl, Decyl, Dodecyl und Stearyl genannt.

Sind $R_1$ und $R_2$ substituierte Alkylgruppen, so können diese verzweigt oder unverzweigt sein und weisen vorzugsweise 1-18, insbesondere 1-12, und besonders bevorzugt 106 C-Atome, und nicht-wasserlöslichmachende Substituenten auf. Beispiele hierfür sind Fluor, Chlor, Cyano, $C_1$-$C_6$-Alkoxy, unsubstituiertes oder durch Chlor, $C_1$-$C_4$-Alkyl oder $C_1$-$C_4$-Alkoxy substituiertes Phenoxy, Naphthoxy, $C_2$-$C_6$-Carbalkoxy

oder heterocyclische aromatische Reste, wie z.B. 2-Thienyl, 2-Benzoxazolyl, 2-Benzimidazolyl, 2-, 3- oder 4-Pyridyl, 2-, 4- oder 6-Chinolyl. Beispiele von substituierten Alkylgruppen sind Trifluormethyl, Trifluoräthyl, Cyanmethyl oder Methoxycarbonylmethyl.

Alkenyl- und Alkinylgruppen können verzweigt oder unverzweigt sein und weisen vorzugsweise 2 bis 18, insbesondere 2 bis 6 C-Atome, auf. Beispiele von Alkenyl- und Alkinylgruppen sind Aethylenyl, Propylenyl, Butenyl, Isobutenyl, Isoprenyl, 1-Pentenyl, 2-Methyl-2-butenyl, 3-Methyl-1-butenyl, 3-Propyl-1-hexenyl, Propadienyl, 1,2-Butadienyl, Aethinyl, Propargyl, 1-Butinyl und 4-Methyl-2-pentinyl.

Bedeutet R $C_1$-$C_4$-Alkyl, so handelt es sich beispielsweise um Methyl, Aethyl, n-Propyl oder n-Butyl.

Bedeuten A, B, $R_1$ und $R_2$ Arylgruppen, so handelt es sich dabei beispielsweise um mono- bis tetracyclische, insbesondere mono- oder bicyclische Reste, also beispielsweise Phenyl, 4-Biphenylyl oder Naphthyl, insbesondere aber um unsubstituiertes oder durch nichtwasserlöslichmachende Substituenten substituiertes Phenyl. Nichtwasserlöslichmachende Substituenten sind beispielsweise Halogen, wie Chlor, Brom oder Fluor, $C_1$-$C_6$-Alkyl, wie Methyl, Aethyl, Isopropyl oder tert.-Butyl, $C_1$-$C_6$-Alkoxy, wie Methoxy oder Aethoxy, $C_1$-$C_6$-Alkylmercapto, wie Methylmercapto, Trifluormethyl, Cyan, Dimethylamino, Diäthylamino, $C_2$-$C_6$-Alkoxycarbonyl, wie Methoxycarbonyl oder Aethoxycarbonyl, Acetylamino, Carbamoyl oder Sulfamoyl.

Bedeutet R Aryl, so handelt es sich dabei beispielsweise um eine Phenyl- oder Naphthylgruppe, die gegebenenfalls durch die oben erwähnten nichtwasserlöslichmachenden Substituenten substituiert sein kann.

Bedeuten A, B, $R_1$, $R_2$, $R_3$, $R_4$ und $R_5$ Aralkylgruppen, dann vorzugsweise solche, die eine 1-12, vorzugsweise 1-6 und insbesondere 1-4 C-Atome enthaltende, verzweigte oder unverzweigte Alkylkette und einen vorzugsweise mono- oder bicyclischen Arylrest enthalten. Als Beispiele seien Benzyl und Phenyläthyl genannt.

Sind $R_1$ und $R_2$ Cycloalkyl, so handelt es sich dabei beispielsweise um Cyclopentyl oder Cyclohexyl.

Bedeuten A und B heterocyclische aromatische Reste, dann vorzugsweise mono- bis tricyclische. Diese können rein heterocyclisch sein oder einen heterocyclischen Ring und einen oder mehrere ankondensierte Benzolringe enthalten, wie z.B. Pyridyl, Pyrimidyl, Pyrazinyl, Triazinyl, Furyl, Pyrrolyl, Thienyl, Chinolyl, Cumarinyl, Benzfuranyl, Benzimidazolyl oder Benzoxazolyl. Die heterocyclischen aromatischen Reste können übliche Substituenten aufweisen, wie sie beispielsweise in der EP Patentschrift Nr. 0061426 erwähnt sind.

$R_1$ und $R_2$ als Alkylcarbamoyl können verzweigtes oder unverzweigtes Alkylcarbamoyl sein, wobei die Alkylgruppen vorzugsweise 1 bis 18, insbesondere 1 bis 12, vor allem 1 bis 8 und besonders bevorzugt 1 bis 4 C-Atome aufweisen. Als Beispiele seien genannt: N-Methylcarbamoyl, N-Aethylcarbamoyl und N,N-Dimethylcarbamoyl.

Stellen $R_1$ und $R_2$ Arylcarbamoyl dar, so handelt es sich dabei beispielsweise um N-Phenylcarbamoyl und N-$\alpha$-Naphthylcarbamoyl.

Bedeuten $R_1$ und $R_2$ Alkoxycarbonyl, so handelt es sich dabei insbesondere um $C_2$-$C_{13}$-Alkoxycarbonylgruppen. Als Beispiele seien genannt: Methoxy-, Aethoxy-, n-Propoxy-, n-Butoxy- und n-Hexyloxycarbonyl.

Sind $R_1$ und $R_2$ Aroylgruppen, so handelt es sich dabei beispielsweise um Benzoyl, 1- oder 2-Naphthoyl, insbesondere aber um durch Halogen, wie Chlor oder Brom, $C_1$-$C_{12}$-Alkyl, $C_1$-$C_{12}$-Alkoxy, Trifluormethyl oder Nitro substituiertes Benzoyl.

Sind $R_1$ und $R_2$ Alkanoylgruppen, so handelt es sich dabei beispielsweise um $C_2$-$C_5$-Alkanoyl, wie Methyl-, Aethyl-, n-Propyl- und tert.-Butylcarbonyl.

Bedeuten $R_3$, $R_4$ und $R_5$ Alkylreste, so handelt es sich dabei um die gleichen Alkylgruppen, die oben bereits für die entsprechende Definition von A, B, $R_1$ und $R_2$ angegeben wurden.

Sind $R_3$, $R_4$ und $R_5$ Aryl, so handelt es sich dabei beispielsweise um Phenyl oder Naphthyl, insbesondere um unsubstituiertes oder durch Halogen, wie Chlor oder Brom, $C_1$-$C_6$-Alkyl oder $C_1$-$C_6$-Alkoxy substituiertes Phenyl.

Bilden $R_4$ und $R_5$ zusammen mit dem N-Atom einen 5- oder 6-gliedrigen heterocyclischen Rest, so handelt es sich dabei beispielsweise um einen Morpholin- oder Piperidinrest.

Als Beispiele für $N^+H(R_3)(R_4)(R_5)$ seien genannt:

$N^+H_4$, $N^+H_3CH_3$, $N^+H_2(CH_3)_2$, $N^+H_3C_2H_5$, $N^+H_2(C_2H_5)_2$, $N^+H_3C_3H_7$-iso, $N^+H_3$-Cyclohexyl, $N^+H_2$-(Cyclohexyl)$_2$, $N^+H_2(CH_3)(C_6H_5)$, $N^+H_3C_6H_5$,$N^+H_3$-para-Toluidin und $N^+H_3$-Benzyl.

Als Beispiele für -N($R_4$)($R_5$) seien genannt: $-NHCH_3$, $-N(CH_3)_2$, $-NCH_2H_5$, $-N(C_2H_5)_2$, -NH-Cyclohexyl, $-NC_6H_5$ und $-NHCH_2C_6H_5$.

Die Gruppen der Formel

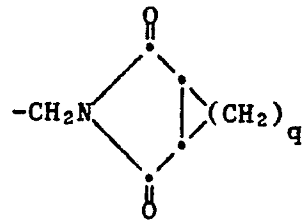

leiten sich von aliphatischen Dicarbonsäuren ab, wie beispielsweise Bernsteinsäure oder 1,2-Cyclobutandicarbonsäure.

Stellt L eine Gruppe der Formel

$$\frac{M^{+n}}{.n}$$

dar, so handelt es sich dabei beispielsweise um ein Alkali-, ein Erdalkali- oder ein Uebergangsmetallkation, insbesonders aber um $Na^{+}$, $K^{+}$, $Mg^{2+}$, $Ca^{2+}$, $Sr^{2+}$, $Ba^{2+}$, $Mn^{2+}$, $Zn^{2+}$, $Cu^{2+}$, $Ni^{2+}$, $Cd^{2+}$, $Co^{3+}$, $Al^{3+}$ und $Cr^{3+}$.

Die Komponente b) der Stoffkompositionen der Formel I kann bis zu vier Gruppen $-SO_3L$, $-CO_2L$, $-PO_3(L)_2$, $-N(R_4)(R_5)$,

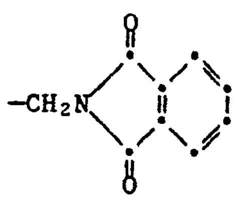 , 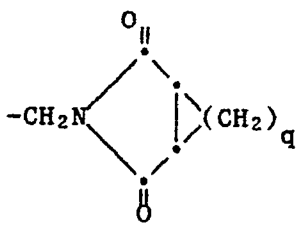 , 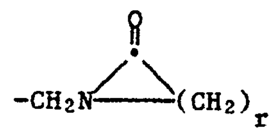

oder -NHCOR, bevorzugt aber eine dieser Gruppen, pro Rest A, B, $R_1$ und $R_2$ aufweisen. Bevorzugt sind die Gruppen der Formeln $-SO_3L$ und $-N(R_4(R_5)$, ganz bevorzugt aber die Gruppe der Formel $-SO_3L$.

Von besonderem Interesse sind Stoffkompositionen enthaltend

a) ein Pyrrolo-pyrrol der Formel II

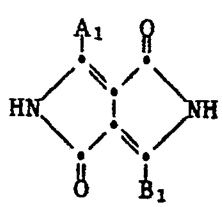 (II),

worin $A_1$ und $B_1$ unabhängig voneinander Arylreste darstellen, und

b) ein Pyrrolo-pyrrol der Formel III

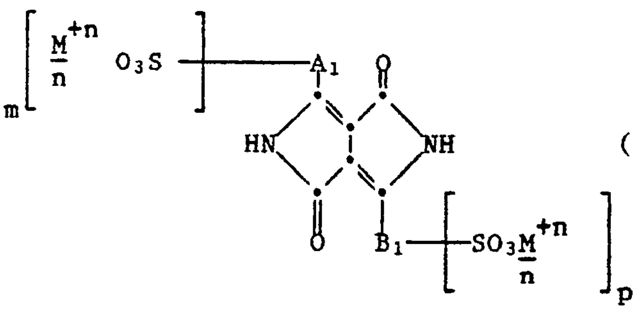

(III),

worin $A_1$ und $B_1$ die oben angegebene Bedeutung haben, M ein Alkali-, Erdalkali- oder Uebergangsmetallkation, n die Zahlen 1, 2 oder 3, und m und p unabhängig voneinander die Zahlen 0, 1, 2, 3 oder 4 bedeuten, wobei aber die Summe m und p mindestens 1 ist.

Bevorzugtes Aryl als $A_1$ und $B_1$ in den oben angegebenen Formeln II und III ist unsubstituiertes oder durch nichtwasserlöslichmachende Substituenten substituiertes Phenyl. Als nichtwasserlöslichmachende Substituenten können die gleichen Beispiele berücksichtigt werden, die bei der entsprechenden Definition für die Reste A und B bereits oben angegeben wurden.

Besonders bevorzugte Stoffkompositionen enthalten ein Pyrrolo-pyrrol der Formel II und ein Pyrrolo-pyrrol der Formel III, wobei $A_1$ und $B_1$ Phenyl oder p-Chlorphenyl, und m und p die Zahlen 0 oder 1 sind, wobei die Summe m und p mindestens 1 ist, n die Zahlen 1 oder 2 und M $Na^+$, $K^+$, $Mg^{2+}$, $Ca^{2+}$, $Sr^{2+}$ oder $Ba^{2+}$ bedeuten, wobei sich der Sulfonsäurerest auf dem Phenylkern in para-Stellung zum Diketopyrrolopyrrolgerüstbefindet.

Die erfindungsgemässen Stoffkompositionen können durch Mischen der einzelnen Pyrrolo-pyrrol-Komponenten a) und b) im gewünschten Mengenverhältnis oder aber auch durch teilweise Sulfonierung, Alkylierung oder Acylierung der Pyrrolo-pyrrol-Komponente a) erhalten werden.

Die Komponenten b) der erfindungsgemässen Stoffkompositionen enthaltend mindestens eine $-SO_3L$-Gruppe können nach an und für sich bekannten Verfahren hergestellt werden, beispielsweise durch Sulfonierung der Pyrrolo-pyrrol-Komponente a) mittels Oleums, Schwefelsäure, flüssigen Schwefeltrioxids oder Chlorsulfonsäure, wobei mindestens eine der Gruppen A, B, $R_1$, $R_2$, $A_1$ und $B_1$ eine sulfonierbare Stelle aufweisen muss. Die einzusetzende Konzentration des Sulfonierungsmittels und die auszuwählenden Reaktionsbedingungen hängen eng mit der Anzahl der Sulfonsäure-Gruppen zusammen, welche in das Pyrrolo-pyrrolmolekül eingeführt werden sollen. Die so erhaltenen Produkte werden anschliessend durch Umsetzung mit einem erfindungsgemäss in Frage kommenden Metallsalz, wie Acetat, Carbonat, Chlorid, Nitrat oder Sulfat, oder mit einem Amin in die entsprechenden Metall- bzw. Aminsalzderivate übergeführt.

Die Pyrrolo-pyrrole gemäss der Komponente a) der erfindungsgemässen Stoffkompositionen stellen bekannte Produkte dar und können beispielsweise gemäss der US-Patentschrift Nr. 4579949 hergestellt werden.

Die Komponenten b) der erfindungsgemässen Stoffkompositionen enthaltend mindestens eine $-CO_2L$- oder $-PO_3(L)_2$-Gruppe können beispielsweise aus den entsprechenden, Carboxyl-, Carbonitril-, Carbonsäureester-, Phosphonsäure- oder Phosphonsäureester-Gruppen enthaltenden Pyrrolo-pyrrolderivaten erhalten werden, wobei die Nitrilgruppen bzw. die Estergruppen nach üblichen Verseifungsmethoden in die freie(n) Säure(n) übergeführt werden. Diese Säuren können dann durch Umsetzung mit den erfindungsgemäss in Frage kommenden Metallsalzen oder Aminen in ihre Metall- bzw. Aminsalze übergeführt werden. Die hierfür benötigten Ausgangsprodukte können beispielsweise aus einem Nitrilderivat enthaltend zusätzlich noch eine Nitril-, Carbonsäure-, Carbonsäureester-, Phosphorsäure- oder Phosphorsäureestergruppe durch Umsetzung mit einem Bernsteinsäureester in Gegenwart einer starken Base gemäss der EP Patentanmeldung Nr. 0094911 hergestellt werden.

Die Komponenten b) enthaltend Carbonsäureamid- oder Dicarbonsäureimidgruppen können ihrerseits beispielsweise durch Umsetzung der Pyrrolo-pyrrol-Komponenten a) mit Formaldehyd und einem Carbonsäureamid oder Dicarbonsäureimid oder mit Hydroxymethylen-N-carbonsäureamid oder -N-dicarbonsäureimid gewonnen werden.

Die Mengenverhältnisse der Komponenten der erfindungsgemässen Stoffkompositionen können beliebig variieren. Bevorzugte Mengenverhältnisse betragen jedoch 0,1 bis 25 Gew.% der Komponente b) und 99,9 bis 75 Gew.% der Komponente a).

Die erfindungsgemässen Stoffkompositionen können als Pigmente zum Färben von hochmolekularem

organischem Material verwendet werden.

Je nach Verwendungszweck kann man die erfindungsgemässen Stoffkompositionen in eine deckendere oder transparentere Pigmentform überführen.

Wird eine deckendere Pigmentform gewünscht, so erweist sich in der Regel eine thermische Nachbehandlung in Wasser oder in einem organischen Lösungsmittel als zweckmässig, gegebenenfalls unter Druck. Vorzugsweise verwendet man organische Lösungsmittel, wie durch Halogenatome, Alkyl- oder Nitrogruppen substituierte Benzole, wie Xylole, Chlorbenzol, o-Dichlorbenzol oder Nitrobenzol, sowie Pyridinbasen, wie Pyridin, Picolin oder Chinolin, ferner Ketone, wie Cyclohexanon, Alkohole, wie Isopropanol, Butanole oder Pentanole, Aether, wie Aethylenglykolmonomethyl- oder -monoäthyläther, Amide, wie Dimethylformamid oder N-Methylpyrrolidon, sowie Dimethylsulfoxid oder Sulfolan. Man kann die Nachbehandlung auch in Wasser, gegebenenfalls unter Druck, in Gegenwart von organischen Lösungsmitteln und/oder mit Zusatz von oberflächenaktiven Substanzen durchführen.

Hochmolekulare organische Materialien, die mit den erfindungsgemässen Stoffkompositionen gefärbt bzw. pigmentiert werden können, sind z.B. Celluloseäther und -ester, wie Aethylcellulose, Nitrocellulose, Celluloseacetat, Cellulosebutyrat, natürliche Harze oder Kunstharze, wie Polymerisationsharze oder Kondensationsharze, wie Aminoplaste, insbesondere Harnstoff- und Melamin-Formaldehydharze, Alkydharze, Phenoplaste, Polycarbonate, Polyolefine, Polystyrol, Polyvinylchlorid, Polyamide, Polyurethane, Polyester, Gummi, Casein, Silikon und Silikonharze, einzeln oder in Mischungen.

Die erwähnten hochmolekularen organischen Verbindungen können einzeln oder in Gemischen als plastische Massen, Schmelzen oder in Form von Spinnlösungen, Lacken, Anstrichstoffen oder Druckfarben vorliegen. Je nach Verwendungszweck erweist es sich als vorteilhaft, die erfindungsgemässen Stoffkompositionen als Toner oder in Form von Präparaten einzusetzen. Bezogen auf das zu pigmentierende hochmolekulare organische Material kann man die erfindungsgemässen Stoffkompositionen in einer Menge von 0,01 bis 30 Gew.%, vorzugsweise von 0,1 bis 10 Gew.%, einsetzen.

Die Pigmentierung der hochmolekularen organischen Substanzen mit den erfindungsgemässen Stoffkompositionen erfolgt beispielsweise derart, dass man eine solche Stoffkomposition gegebenenfalls in Form von Masterbatches diesen Substraten under Verwendung von Walzwerken, Misch- oder Mahlapparaten zumischt. Das pigmentierte Material wird hierauf nach an sich bekannten Verfahren, wie Kalandrieren, Pressen, Strangpressen, Streichen, Giessen oder Spritzguss in die gewünschte endgültige Form gebracht. Oft ist es erwünscht, zur Herstellung von nicht starren Formlingen oder zur Verringerung ihrer Sprödigkeit den hochmolekularen Verbindungen vor der Verformung sogenannte Weichmacher einzuverleiben. Als solche können z.B. Ester der Phosphorsäure, Phthalsäure oder Sebacinsäure dienen. Die Weichmacher können vor oder nach der Einverleibung der erfindungsgemässen Stoffkomposition in die Polymeren eingearbeitet werden. Es ist ferner möglich, zwecks Erzielung verschiedener Farbtöne den hochmolekularen, organischen Stoffen neben den erfindungsgemässen Stoffkompositionen noch Füllstoffe bzw. andere farbgebende Bestandteile, wie Weiss-, Bunt- oder Schwarzpigmente, in beliebigen Mengen zuzufügen.

Zum Pigmentieren von Lacken und Druckfarben werden die hochmolekularen organischen Materialien und die erfindungsgemässen Stoffkompositionen gegebenenfalls zusammen mit Zusatzstoffen, wie Füllmitteln, anderen Pigmenten, Siccativen oder Weichmachern, in einen gemeinsamen organischen Lösungsmittel oder Lösungsmittelgemisch fein dispergiert bzw. gelöst. Man kann dabei so verfahren, dass man die einzelnen Komponenten für sich oder auch mehrere gemeinsam dispergiert bzw. löst, und erst hierauf alle Komponenten zusammenbringt.

Die erhaltenen Färbungen, beispielsweise in Kunststoffen, Fasern, Lacken oder Drucken, zeichnen sich durch gute allgemeine Eigenschaften, wie hohe Farbstärke, gute Dispergierbarkeit, gute Ueberlackier-, Migrations-, Hitze-, Licht- und Wetterbeständigkeit, sowie durch einen guten Glanz aus.

Ausserdem weisen die erfindungsgemässen Stoffkompositionen im Vergleich zu den unsulfonierten, uncarboxylierten, unphosphonierten oder nicht N-imidomethylierten Pyrrolo-pyrrolen eine verbesserte Rheologie insbesonders in Lacken und Druckfarben, sowie eine verbesserte Hitzebeständigkeit und Verzugseigenschaft in der Applikation auf.

Ein weiterer Gegenstand der vorliegenden Erfindung sind neue Verbindungen der Formel IV

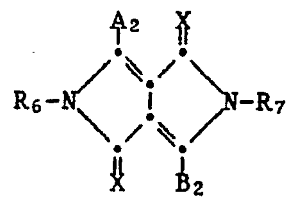

(IV),

enthaltend mindestens eine Gruppe der Formeln $-SO_3L$, $-CO_2L$, $-PO_3(L)_2$,

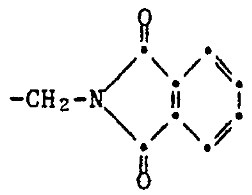, 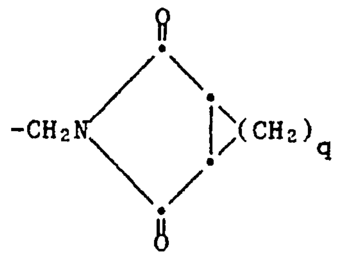, 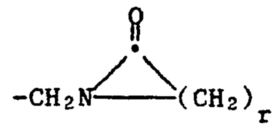

oder -NHCOR, worin $A_2$ und $B_2$ gleiche oder voneinander verschiedene Alkyl-, Aralkyl-, Aryl- oder heterocyclische aromatische Reste, $R_6$ und $R_7$ H-Atome, unsubstituierte oder substituierte Alkylreste, ferner Alkenyl-, Alkinyl-, Aralkyl-, Cycloalkyl-, Benzyl-, Carbamoyl-, Alkylcarbamoyl-, Arylcarbamoyl-, Alkoxycarbonyl-, Aryl-, Alkanoyl- oder Aroylgruppen, und X ein O- oder S-Atom bedeuten, L -H oder eine Gruppe der Formeln

$$\frac{M^{+n}}{n}$$

oder $N^+H(R_8)(R_9)(R_{10})$ darstellt, M ein ein-, zwei- oder dreiwertiges Metallkation, n die Zahlen 1, 2 oder 3 und $R_8$, $R_9$ und $R_{10}$ unabhängig voneinander -H, Alkyl-, Aralkyl-, $C_5$-$C_6$-Cycloalkyl-oder Arylreste bedeuten oder $R_9$ und $R_{10}$ zusammen mit dem N-Atom einen 5- oder 6-gliedrigen heterocyclischen Rest bilden, q die Zahlen 0 bis 4, r die Zahlen 3 bis 5 und R Aryl oder $C_1$-$C_4$-Alkyl darstellen, wobei L kein Natriumkation bedeuten darf, wenn die Verbindungen der Formel IV eine $-SO_3L$-Gruppe aufweist.

Für die Ausdeutung bzw. Bevorzugung der verschiedenen Symbole $A_2$, $B_2$, R, $R_6$ bis $R_{10}$, X, L, M, n, q und r sind die gleichen Definitionen zu berücksichtigen, die für die entsprechenden Gruppen A, B, R, $R_1$ bis $R_5$, X, L, M, n, q und r bereits aufgeführt wurden.

Von besonderem Interesse sind Verbindungen der Formel V

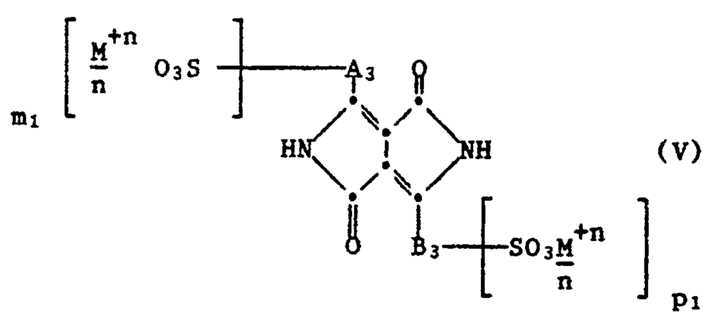

(V),

worin $A_3$ und $B_3$ Phenyl oder Naphthyl und $m_1$ und $p_1$ die Zahlen 0 oder 1 bedeuten, wobei die Summe $m_1$ und $p_1$ mindestens 1 ist, ferner n die Zahlen 1, 2 oder 3 und M ein Erdalkali- oder Uebergangsmetallkation, $Li^+$ oder $K^+$ bedeuten.

Von ganz besonderem Interesse sind Verbindungungen der Formel V, worin $A_3$ und $B_3$ Phenyl und $m_1$ und $p_1$ die Zahlen 0 oder 1 bedeuten, wobei die Summe $m_1$ und $p_1$ mindestens 1 ist, n die Zahlen 1 oder 2 und M $K^+$, $Ca^{2+}$, $Mg^{2+}$, $Sr^{2+}$, $Ba^+$, $Zn^{2+}$ oder $Cd^{2+}$ bedeuten.

Die Verbindungen der Formeln IV und V können nach den gleichen Herstellungsmethoden gewonnen werden, welche zur Herstellung der eingangs erwähnten Komponenten b) der Stoffkompositionen der Formel I bereits aufgeführt wurden.

Die Verbindungen der Formeln IV und V stellen wertvolle neue Zwischenprodukte dar, die beispielsweise für die Synthese von weiteren Pyrrolo-pyrrolen verwendet werden können, so beispielsweise zur Herstellung von Säureamiden und Säureester-derivaten aus den entsprechenden Sulfon-, Carbon- oder Phosphonsäurederivaten.

Die Verbindungen der Formeln IV und V können aber auch als Pigmente zum Einfärben der oben aufgeführten hochmolekularen organischen Materialien eingesetzt werden.

Hierfür können sie als Rohprodukte oder aber nach zweckmässiger Konditionierung/Nachbehandlung, beispielsweise wie oben für die erfindungsgemässen Stoffkompositionen bereits aufgeführt, eingesetzt werden.

In den nachfolgenden Beispielen bedeuten die Prozente Gewichtsprozente.

Beispiel 1:

6 g 1,4-Diketo-3,6-diphenylpyrrolo-[3,4-c]-pyrrol werden im Laufe von 10 Minuten unter Rühren bei 10-15 °C in 78 g rauchender Schwefelsäure (25 % $SO_3$) eingetragen. Die resultierende Mischung wird während 3 Stunden bei 15-20 °C gerührt und darauf auf 800 g Eis ausgetragen. Nach 16stündigem Stehenlassen bei 20 °C wird die entstandene Suspension filtriert, und das erhaltene dunkelrote Produkt wird nach dreimaligem Waschen mit wenig Wasser in etwa 700 g Wasser unter Zusatz einer Ammoniaklösung bei 80 °C gelöst. Die erhaltene kirschrote Lösung wird bei 80 °C unter Rühren mit 45 g Ammoniumchlorid versetzt und während 30 Minuten bei 80 °C nachgerührt. Der ausgefallene Niederschlag wird bei 60 °C abfiltriert, mit einer 2%igen Ammoniumchloridlösung gewaschen und bei 100 °C getrocknet. Man erhält 7,6 g eines dunkelroten Produktes, das im wesentlichen das Ammoniumsalz der 1,4-Diketo-3,6-diphenyl-pyrrolo-[3,4-c]-pyrrol-4'-monosulfonsäure darstellt. Dieses Produkt löst sich in kaltem Wasser mit kirschroter Farbe; beim Sieden dieser wässrigen Lösung tritt ein Farbumschlag nach orangerot ein. Beim Abkühlen der heissen Lösung kehrt die ursprüngliche Farbe zurück.

Beispiel 2:

10 g 1,4-Diketo-3,6-diphenylpyrrolo-[3,4-c]-pyrrol werden im Laufe von 10 Minuten unter Rühren bei 5-10 °C in 150 g rauchender Schwefelsäure (12,5 % $SO_3$) eingetragen. Die erhaltene Mischung wird während 16 Stunden bei 20 °C gerührt und darauf auf 700 g Eis ausgetragen. Die erhaltene Lösung wird mit Wasser auf 1100 g gestellt und bei 75 °C unter Rühren mit 60 g Natriumchlorid versetzt. Die Farbe der resultierenden Suspension wandelt sich in kurzer Zeit von orange nach rot um. Eine Tupfprobe zeigt farblosen Auslauf. Nach halbstündigem Rühren bei 70-75 °C wird der Niederschlag abfiltriert und mit einer 5%igen Natriumchloridlösung neutral gewaschen. Nach zweimaligem Waschen mit Wasser wird das feuchte Produkt in Wasser suspendiert. Die mit Wasser auf 600 g gestellte Suspension wird bei 90-95 °C innerhalb von 20 MInuten mit einer Lösung von 25 g Bariumchlorid ($BaCl_2 \cdot 2H_2O$) in 100 g Wasser versetzt und während einer Stunde im Sieden gehalten. Der entstandene Niederschlag wird abfiltriert, 6-mal mit kaltem Wasser gewaschen und dann gefriergetrocknet. Man erhält 19,4 g eines dunkelroten Produktes, welches im wesentlichen das Bariumsalz der 1,4-Diketo-3,6-diphenylpyrrolo-[3,4-c]-pyrrol-4',4''-disulfonsäure darstellt. Wasserbestimmung (Gewichtsverlust bei 130 °C): 3,2 %;

| | | | |
|---|---|---|---|
| Korrigierte Analysenwerte: | gef.: Na 0,51 % | ber.: --- |
| | Ba 22,0 % | 23,53 % |
| | S 11,0 % | 10,98 % . |

Das erhaltene Produkt färbt Lacke und Kunststoffe in lichtbeständigen roten Tönen. Seine Hitzebeständigkeit in HDPE ist vorzüglich.

Beispiel 3:

In analoger Weise wie in Beispiel 2 beschrieben erhält man das Calciumsalz der 1,4-Diketo-3,6-diphenylpyrrolo-[3,4-c]-pyrrol-4′,4″-disulfonsäure;

Ausbeute: 16,7 g.

Analyse: Wasser, bei 150 ° C ausgetrieben, Gewichtsverlust: 6,2 %;
Ca korr. gef.: 7,94 % ber.: 8,42 %;
S korr. gef.: 12,42 % ber.: 13,18 %.

Das erhaltene Produkt färbt Lacke und PVC in lichtbeständigen roten n und mit sehr guter Migrationsbeständigkeit.

Beispiel 4:

70 g 1,4-Diketo-3,6-diphenylpyrrolo-[3,4-c]-pyrrol werden im Laufe von 20 Minuten bei 5-12 ° C in 1050 g rauchender Schwefelsäure (12,5 % $SO_3$) eingetragen. Nach 16stündigem Rühren bei 20 ° C wird das Sulfierungsgemisch unter Rühren auf Eis ausgetragen, Endvolumen 9000 ml. Die entstandene Lösung wird bei 22 ° C mit 450 g Natriumchlorid versetzt. Es entsteht zunächst ein orangefarbiger Niederschlag. Die Suspension wird nun auf 75 ° C erhitzt, die Farbe der Suspension ändert sich hierbei nach rot. Nach 30 minütigem Rühren bei 75 ° C wird die Suspension filtriert, und das Nutschgut wird dann dreimal mit Wasser gewaschen. Das feuchte Produkt wird in Wasser suspendiert, mit Wasser auf 1500 g eingestellt und die erhaltene Suspension wird unter Rühren bei 75 ° C mit einer Natriumhydroxidlösung auf pH 7 eingestellt. Nach halbstündigem Rühren wird die Suspension abermals filtriert, der Rückstand wird zweimal mit kaltem Wasser gewaschen und bei 120 ° C im Vacuum getrocknet. Man erhält 113 g des Natriumsalzes der 1,4-Diketo-3,6-diphenylpyrrolo-[3,4-c]-pyrrol-4′,4″-disulfonsäure in Form eines dunkelroten Produktes.

Beispiel 5:

20 g 1,4-Diketo-3,6-diphenylpyrrolo-[3,4-c]-pyrrol werden bei 5-10 ° C unter Rühren in 300 g rauchender Schwefelsäure (12,5 % $SO_3$) eingetragen. Nach 19stündigem Rühren bei 20 ° C wird das Reaktionsgemisch auf Eis ausgetragen, und die Mischung wird mit Wasser auf 2000 g gestellt. Dann lässt man unter Rühren bei 20 ° C eine Lösung von 8,05 g Hexamethylendiamin in 200 g Wasser und 8,3 g Ameisensäure im Laufe von 20 Minuten zutropfen. Anfänglich bildet sich eine Trübung, gegen Schluss der Zugabe beginnt ein Niederschlag auszufallen. Nach 5stündigem Rühren bei 20-25 ° C wird die Suspension filtriert und mit kaltem Wasser neutral gewaschen. Nach Trocknung bei 90 ° C im Vakuum erhält man 33,5 g des Hexamethylendiaminsalzes der 1,4-Diketo-3,6-diphenylpyrrolo-[3,4-c]-pyrrol-4′,4″-disulfonsäure.

Analyse: ber. für $C_{24}H_{28}N_4O_8S_2$: C 51,05 H 5,00 N 9,92 S 11,36%;
gef.: C 50,18 H 5,12 N 9,65 S 11,23%.

Auf analoge Weise lassen sich die Di-o-tolylguanidin-, Dicyclohexylamin- und $C_{12}H_{25}$- bzw. $C_{18}H_{37}$-/$C_{22}H_{45}$-Aminsalze der 1,4-Diketo-3,6-diphenylpyrrolo-[3,4-c]-pyrrol-4′,4″-disulfonsäure herstellen.

Beispiel 6:

20 g 1,4-Diketo-3,6-diphenylpyrrolo-[3,4-c]-pyrrol werden bei 5-10 ° C unter Rühren in 300 g rauchender Schwefelsäure (12,5 % $SO_3$) eingetragen. Nach 19stündigem Rühren bei 20 ° C wird das Reaktionsgemisch auf Eis ausgetragen, und die Mischung wird mit Wasser auf 2000 g gestellt. Zur erhaltenen Lösung lässt man im Laufe von 10 Minuten eine Lösung von 14,85 g 4-Toluidin in 70 g 100%iger Essigsäure zutropfen. Während des Zulaufs der 4-Toluidinlösung fällt ein Niederschlag aus. Nach 4stündigem Rühren bei 20 ° C wird der orangerote Niederschlag abfiltriert, mit kaltem Wasser neutral gewaschen und bei 90 ° C im Vacuum getrocknet. Man erhält 42,9 g des 4-Toluidinsalzes der 1,4-Diketo-3,6-diphenylpyrrolo-[3,4-c]-pyrrol-4′,4″-disulfonsäure.

Analyse: ber.: C 57, 99 H 4,56 N 8,45 S 9,68 %;
gef.: C 57,34 H 4,62 N 8,27 S 9,64 %.

Beispiel 7:

5,1 g des nach Beispiel 4 erhaltenen Natriumsalzes der 1,4-Diketo-3,6-diphenylpyrrolo-[3,4-c]-pyrrol-4′,4″-disulfonsäure werden mit 150 g Wasser und 5 g kristallisiertem Magnesiumsulfat 17 Stunden unter Rühren zum Sieden erhitzt. Die erhaltene heisse Suspension wird filtriert, der Rückstand wird 5-mal mit

heissem Wasser gewaschen und bei 110 °C im Vacuum getrocknet. Man erhält 5 g des entsprechenden Magnesiumsalzes als dunkelrotes Produkt.

Analyse: Mg ber.: 5,15 %;
Mg gef. (korr.): 4,49 %; Na gef. (korr.): 0,5 %;
Wasser gef.: 9,3 %.

Das erhaltene Produkt färbt Lacke und Kunststoffe in roten Tönen mit vorzüglicher Licht- und Migrationsbeständigkeit und zeigt sehr gute Hitzebeständigkeit in HDPE.

In analoger Weise werden die Aluminium-, Zink-, Cadmium-, Kupfer-und Nickelsalze der 1,4-Diketo-3,6-diphenylpyrrolo-[3,4-c]-pyrrol-4',4"-disulfonsäure erhalten. Dieselben eignen sich ebenfalls zur Einfärbung von Lacken und Kunststoffen und weisen allgemeine, gute Pigmenteigenschaften und Hitzebeständigkeit in HDPE auf.

Beispiel 8:

20 g 1,4-Diketo-3,6-diphenylpyrrolo-[3,4-c]-pyrrol werden bei 5-10 °C innerhalb 15 Minuten in 300 g rauchender Schwefelsäure (12,5 % $SO_3$) eingetragen. Nach halbstündigem Rühren bei 15-20 °C werden bei 4-8 °C 17 g Essigsäureanhydrid in 20 Minuten zugetropft. Die erhaltene Mischung wird nun während 21 Stunden bei 20 °C gerührt. 5 Stunden nach Zugabe des Essigsäureanhydrids beginnen sich orange Kristalle abzuscheiden. Am Ende der Reaktion liegt eine Suspension derber Kristalle vor. Unter Kühlung werden bei 10-20 °C im Laufe einer Stunde 100 g 100%iger Essigsäure zugetropft; es tritt wiederum Lösung ein. Die erhaltene Mischung wird während weiterer 24 Stunden bei 20 °C gerührt, die entstandene Suspension wird auf einer Glassinternutsche filtriert, und der Rückstand wird dreimal mit 100%iger Essigsäure gewaschen. Das Produkt wird in 100%iger Essigsäure suspendiert, abermals abfiltriert und fünfmal mit 100%iger Essigsäure gewaschen. Nach Trocknen im Vacuum erhält man 29 g eines dunklen Pulvers, das gemäss Verbrennungsanalyse und NMR-Spektrum der 1,4-Diketo-3,6-diphenylpyrrolo-[3,4-c]-pyrrol-4',4"-disulfonsäure entspricht.

Beispiel 9:

10 g der nach Beispiel 8 erhaltenen freien 1,4-Diketo-3,6-diphenylpyrrolo-[3,4-c]-pyrrol-4',4"-disulfonsäure werden unter Rühren bei 20 °C in 105 g Chlorsulfonsäure eingetragen. Die erhaltene Lösung wird auf 5 °C gekühlt, dann werden 49 g Thionylchlorid in 10 Minuten zugetropft. Die Mischung wird während 3,5 Stunden bei 60 °C verrührt und darauf auf 10 °C gekühlt. Die Reaktionsmischung wird nun unter Rühren auf eine Mischung von 1200 g Eis und 200 g Natriumchlorid ausgetragen. Der ausgefallene Niederschlag wird abfiltriert und mit einer 10%igen Natriumchloridlösung säurefrei gewaschen. Nach dem Trocknen im Vacuum bei 70 °C erhält man das Natriumchlorid enthaltende 1,4-Diketo-3,6-diphenylpyrrolo-[3,4-c]-pyrrol-4',4"-disulfonsäurechlorid in Form eines dunkelroten Pulvers.

Beispiel 10:

14,5 g 1,4-Diketo-3,6-diphenylpyrrolo-[3,4-c]-pyrrol werden bei 0-5 °C im Laufe von 10 Minuten unter Rühren in 290 g 96%iger Schwefelsäure eingetragen. Nach einer Stunde Rühren werden 290 g Schwefelsäure-Monohydrat zugefügt; es tritt alsbald Lösung ein. Bei 5 °C werden 18,1 g N-Hydroxymethylphthalimid in 15 Minuten zugefügt. Man lässt die Temperatur in 3 Stunden auf 14 °C steigen und rührt darauf während 16 Stunden bei 15-20 °C nach. Das erhaltene Gemisch wird sodann auf 3000 g Eiswasser ausgetragen, der ausgefallene bräunlichgelbe Niederschlag wird abfiltriert und mit Wasser säurefrei gewaschen. Nach dem Trocknen bei 80 °C erhält man 29,9 g eines braungelben Rohproduktes. Zur Reinigung wird das pulverisierte Rohprodukt mit 300 ml Aethanol während einer Stunde bei Siedetemperatur verrührt, der Niederschlag wird in der Wärme abfiltriert, mit Aethanol gewaschen und bei 60 °C getrocknet. Man erhält ein braungelbes Produkt, das aus einem Gemisch von Verbindungen der Formel

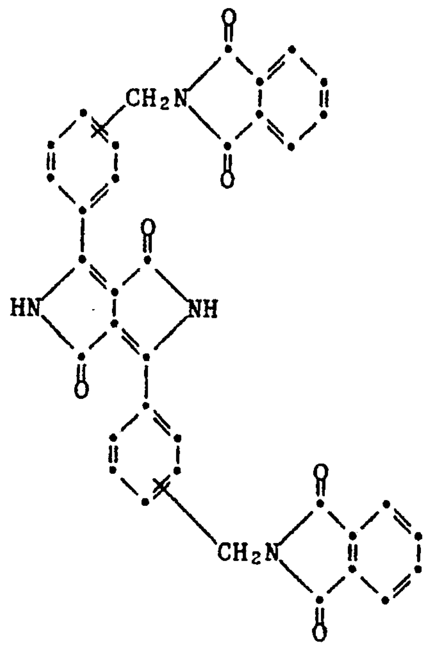

besteht.

Beispiel 11A:

In einem 350 ml Sulfierkolben mit Propellerrührer werden 4,83 g Natrium, 0,1 g Sulfobernsteinsäure-bis-2-äthylhexylester-Na-Salz (als Emulgator) und 85 ml tert.-Amylalkohol vorgelegt, und das Gemisch auf 95-102 °C erwärmt. Unter heftigem Rühren wird das geschmolzene Metall im Alkohol aufgelöst. Die erhaltene Lösung wird auf ca. 90 °C abgekühlt. Zu dieser Lösung werden dann 15,0 g getrocknete 3-Cyanbenzoesäure und anschliessend 11,8 g destillierter Bernsteinsäure-di-tert.-butylester gegeben. Das erhaltene Gemisch wird auf schwachen Rückfluss (ca. 95 °C bis max. 101 °C) erwärmt und bei dieser Temperatur während 6 Stunden weitergerührt. Anschliessend kühlt man auf 35 °C, lässt 85 ml Methanol zutropfen, neutralisiert langsam das Reaktionsgemisch mit 13 g Eisessig, verdünnt noch mit 30 ml Methanol, kocht während 2 Stunden am Rückfluss und filtriert bei 50 °C. Das Produkt wird mit Methanol, dann Aceton und anschliessend mit verdünnter Salzsäure (0,1 N) gewaschen. Nach der Trocknung im Vakuumofen bei 80 °C erhält man ein oranges Produkt der Formel

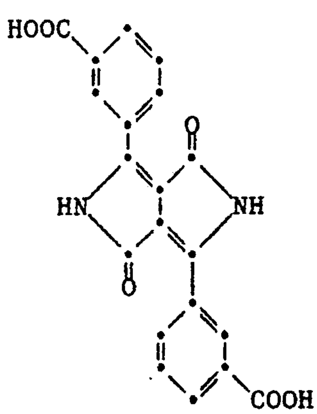

Beispiel 11B:

In einem 200 ml Sulfierkolben mit Propellerrührer werden nacheinander 8,7 g Kalium-tert.-butylat, 55 ml tert.-Butanol, 9,65 g 3-Cyanbenzoesäure-isopropylester (dest.) und 5,6 g destillierter Bernsteinsäurediisopropylester vorgelegt. Das Reaktionsgemisch wird auf Rückfluss erwärmt (ca. 85°C) und während 5 Std. am Rückfluss gehalten. Anschliessend lässt man auf 50°C abkühlen, tropft 55 ml Methanol hinzu, neutralisiert langsam mit einem Gemisch von 6,9 g Eisessig und 5 ml Methanol, und überträgt das ganze Gemisch in einem Becherglas enthaltend 200 ml Methanol und 50 ml Wasser. Nach kurzen Rühren wird das Produkt filtriert, der Rückstand wird mit wässrigem Methanol (1:1) gewaschen und bei 80°C im Vakuumofen getrocknet. Man erhält 4,75 g eines Rohproduktes entsprechend ca. 41,3 % der Theorie.

3,70 g des so erhaltenen Rohproduktes (1,4-Diketo-3,6-diphenylpyrrolo-[3,4-c]-pyrrol-3′,3″-isopropoxycarbonyl) werden in einem Gemisch von 45 ml Dimethylsulfoxid und 15 ml Wasser vorgelegt und mit einer Lösung von 4,67 g 30%iger Natronlauge und 5 ml Wasser versetzt. Nach 2 1/2 Stunden Rühren stellt sich ein pH-Wert von ca. 12 ein. Durch Zulauf von konzentrierter Salzsäure (mit Wasser 1:1 verdünnt) stellt man den pH der Reaktionsmischung auf 1,5 hinunter und heizt dann das Gemisch während 3 Stunden am Rückfluss.

Man kühlt es dann auf 80°C ab, filtriert das erhaltene Produkt und wäscht es nacheinander mit Methanol, Aceton und warmem Wasser. Nach Trocknung im Vakuumofen bei 80°C erhält man die Verbindung der im Beispiel 11A angegebenen Struktur als oranges Pulver.

Beispiel 11C:

0,94 g der Verbindung des Beispiels 11B wird in einem 1:1 Dimethylsulfoxyd-Wasser-Gemisch (60 ml) suspendiert und mit 1,47 g 30%iger Natronlauge versetzt. Die resultierende Suspension wird während 2 Stunden bei 60°C gerührt. Anschliessend gibt man 0,5 ml Eisessig zu, um den pH-Wert auf 8,5 einzustellen. Hierauf gibt man 0,44 g Calciumacetat hinzu und erhitzt das Reaktionsgemisch weiter während 3 Stunden am Rückfluss. Nach Abkühlen auf 60°C verdünnt man die Suspension mit 120 ml Wasser, rührt während 30 Minuten bei dieser Temperatur und filtriert. Das erhaltene Pigment-Salz wird mit heissem Wasser gewaschen und im Vakuumofen bei 80°C über Nacht getrocknet. Auf diese Weise erhält man ein oranges Pulver der folgenden Struktur

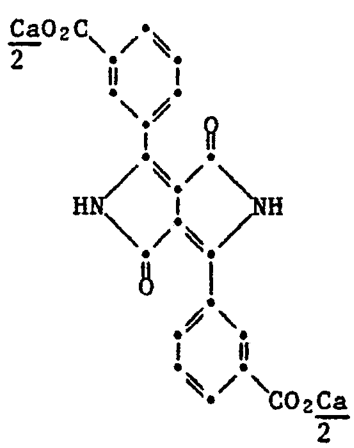

,

das in der Applikation sehr gute Pigmenteigenschaften aufweist.

Beispiel 11D:

Verwendet man gemäss obigem Beispiel 11C 0,607 g Zinkacetat•$2H_2O$ anstelle von Calciumacetat, so erhält man bei sonst analoger Arbeitsweise die Verbindung der Formel

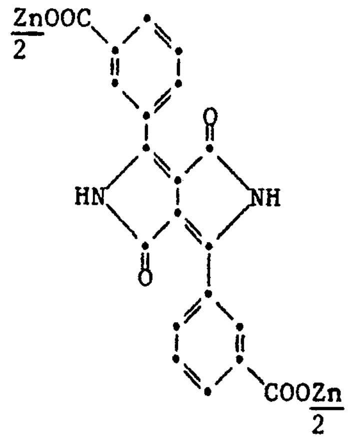

,

die, in PVC eingearbeitet, eine orange Färbung ergibt.

Beispiel 12A:

7,0 g der gemäss Beispiel 4 hergestellten Verbindung werden in 60 g 98%iger Schwefelsäure gelöst, und die Lösung wird mit Wasser auf 2 Liter verdünnt. Anschliessend werden 140 g des gemäss Beispiel 6 der US-Patentschrift Nr. 4579949 hergestellten Pigments als feuchter Presskuchen zugegeben, das Ganze wird mit Wasser auf 4 ℓ ergänzt und während 4 Stunden bei Raumtemperatur gerührt. Hierauf wird das Gemisch auf 75°C geheizt, dann werden 100 g Natriumchlorid innert 30 Minuten eingetragen, und anschliessend wird während 30 Minuten bei dieser Temperatur weitergerührt. Anschliessend kühlt man die Suspension auf Raumtemperatur ab, filtriert ab, wäscht den Rückstand zuerst mit 2 ℓ einer 2%igen Natriumchlorid-Lösung und dann mit Wasser. Man erhält 480 g eines wasserfeuchten Gemisches, das gemäss Beispielen 12B bis 12E weiterverarbeitet wird.

Beispiel 12B:

120 g des gemäss Beispiel 12A erhaltenen wasserfeuchten Gemisches werden unter Vakuum bei ca. 80°C über Nacht getrocknet. Man erhält 36 g einer Produktemischung bestehend aus den Verbindungen der Formeln

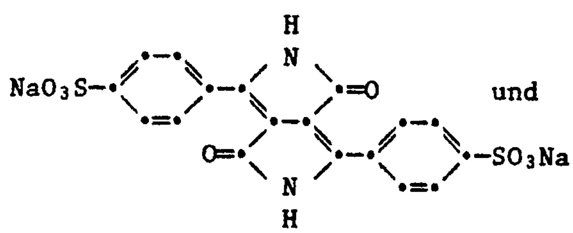

und

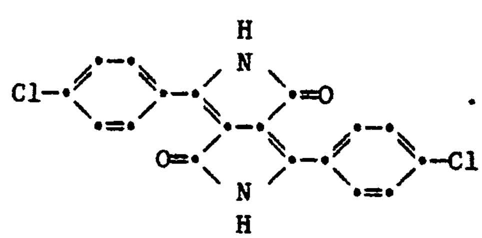

.

Beispiel 12C:

120 g des gemäss Beispiel 12A erhaltenen wasserfeuchten Gemisches werden bis auf ein Volumen von 1500 ml in Wasser dispergiert und auf 90-95°C aufgeheizt. 4,5 g $BaCl_2 \cdot 2H_2O$ werden in wenig Wasser gelöst und innert 30 Minuten zugetropft. Die erhaltene Suspension wird während einer Stunde bei dieser Temperatur nachgerührt, dann auf Raumtemperatur abgekühlt, filtriert, und das erhaltene Produkt wird mit Wasser gewaschen und getrocknet. Man erhält 39,3 g einer Mischung bestehend aus den Verbindungen der Formeln

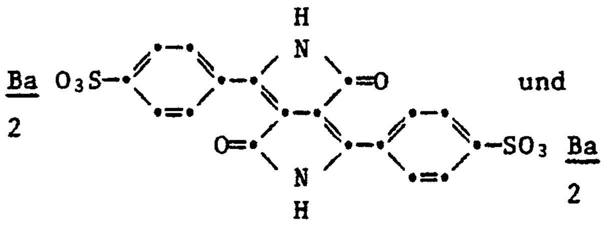

und

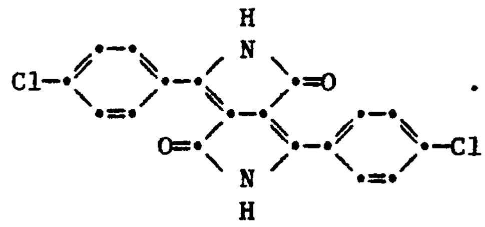

.

Beispiel 12D:

Verwendet man gemäss obigem Beispiel 12C 8 g $Al_2(SO_4)_3 \cdot 18H_2O$ statt $BaCl_2 \cdot 2H_2O$, so erhält man bei sonst analoger Arbeitsweise 34,7 g einer Mischung bestehend aus den Verbindungen der Formeln

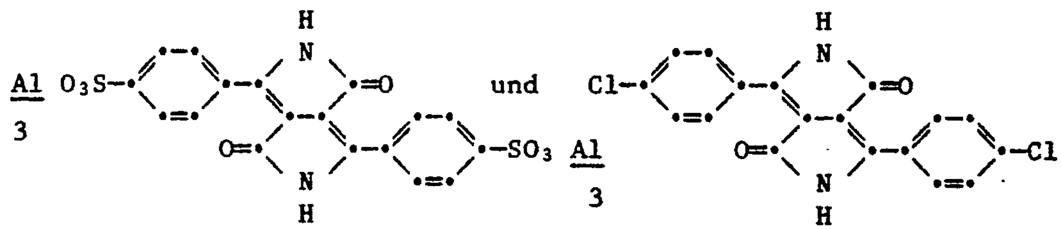

.

Beispiel 12E:

Arbeitet man analog Beispiel 12C, verwendet aber 3 g $MgSO_4$ anstelle von $BaCl_2 \cdot 2H_2O$, so erhält man 35,6 g einer Mischung bestehend aus den Verbindungen der Formeln

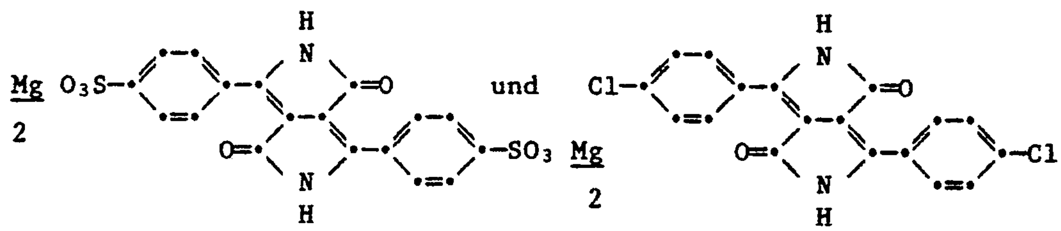

.

Beispiel 13:

1,42 g des gemäss Beispiel 12 der US-Patentschrift Nr. 4579949 hergestellten Pigments werden in 300 ml 98%iger $H_2SO_4$ gelöst und dann mit Wasser auf 1500 ml verdünnt. Die erhaltene Suspension wird am Rückfluss erhitzt. Anschliessend werden 33,2 g der gemäss Beispiel 6 der US-Patentschrift Nr. 4579949 hergestellten Verbindung als feuchter Presskuchen in die resultierende violette Lösung portionsweise eingetragen und während 4 Stunden nachgerührt. Man kühlt unter Rühren auf 60° C ab, filtriert und wäscht neutral das erhaltene Produkt mit Wasser. Nach Vakuumtrocknung bei 80° C über Nacht erhält man 32,7 g einer Mischung bestehend aus den Verbindungen der Formeln

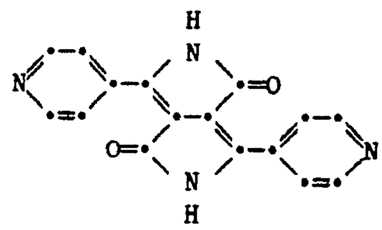

und

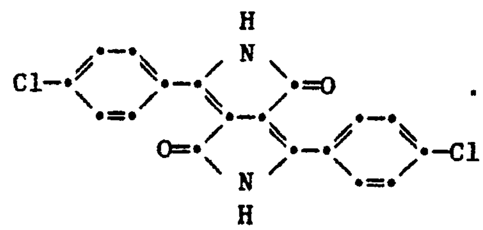

.

Beispiel 14:

1,84 g der gemäss Beispiel 11A hergestellten Verbindung werden mit 300 ml Wasser und 15 ml 30%iger Natronlauge gelöst. Man trägt anschliessend 33 g der gemäss Beispiel 6 der US-Patentschrift Nr. 4579949 hergestellten Verbindung als feuchter Presskuchen ein. Die resultierende Suspension wird während 3 Stunden gerührt, auf 75 °C erhitzt und bei dieser Temperatur innert 30 Minuten mit einer Lösung von 4,5 g $BaCl_2 \cdot 2H_2O$ in 50 ml Wasser versetzt und während 2 Stunden nachgerührt. Nach dem Abkühlen auf Raumtemperatur wird die Suspension filtriert, der Rückstand wird neutral gewaschen und unter Vakuum bei 80 °C über Nacht getrocknet. Man erhält 35,3 g einer Mischung aus den Verbindungen der Formeln

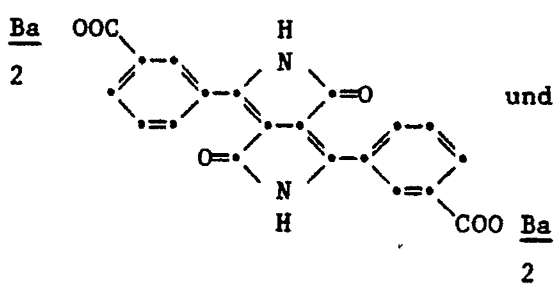

und

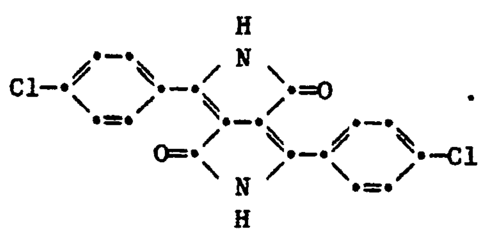

.

Beispiel 15:

Misst man unter streng vergleichbaren Bedingungen die Viskosität einer 6%igen Alkydmelamin-Lackanreibung des gemäss Beispiel 12B erhaltenen Gemisches mit Hilfe eines Rotationsviskosimeters der Firma Brookfield Engineering Laboratories, Stoughton, Mass., USA, so erhält man bei verschiedenen Spindelgeschwindigkeiten (bei 1,5, 6, 30 und 60 U./Min.) für die gemäss Beispiel 12B erhaltene Mischung niedrigere Viskositätswerte als diejenigen des unbehandelten Pigmentes (Beispiel 6 der US-PS Nr. 4579949) und damit ein günstigeres rheologisches Verhalten in der Applikation.

Beispiel 16:

In einem 100 ml Sulfierkolben mit Propellerrührer werden unter Argon nacheinander 6,94 g Kalium-tert.butylat, 35 ml tert.-Butanol, 10,63 g rohes 4-Cyanphosphonsäure-diäthylester und 4,09 g Bernsteinsäure-diisopropylester vorgelegt. Das Reaktionsgemisch wird auf 78-81 °C (Rückfluss) erwärmt und während 4,5 Std. bei dieser Temperatur gehalten. Anschliessend lässt man das Reaktionsgemisch auf 50 °C abkühlen, giesst es in einen 50 ml Tropftrichter und tropft es innert 10 Min. auf 100 ml Eiswasser. Nach kurzem Ausrühren wird die Suspension mit 22 ml konz. Salzsäure (ca. 18 %) versetzt, bis der pH-Wert 1,5 beträgt. Dann wird die erhaltene Suspension bei Raumtemperatur gerührt und filtriert. Das erhaltene Produkt wird mit heissem Wasser gewaschen und bei 80 °C im Vakuumofen getrocknet. Man erhält 3,11 g eines Produktes, das im wesentlichen aus einem Gemisch der Verbindungen folgender Formel entspricht

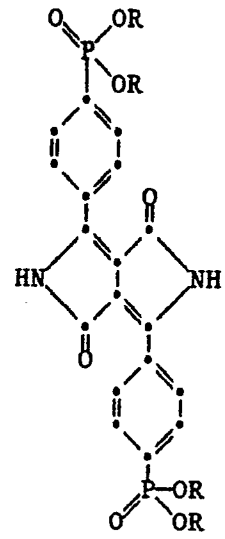

,

wobei R Aethyl, Isopropyl und tert.-Butyl darstellt. Dieses Gemisch lässt sich in einem Wasser-Dimethylformamidgemisch (1:1) mittels Salzsäure zu den entsprechenden Phosphorsäure-monoesterderivaten der Formel

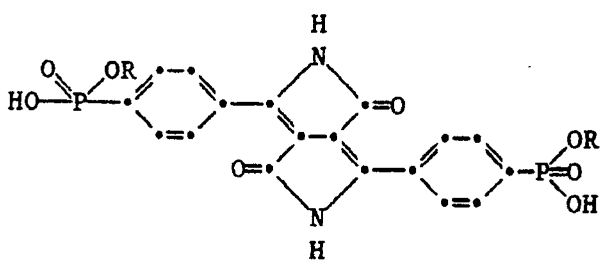

umwandeln,

wobei R die oben angegebene Bedeutung hat.

Beispiel 17:

Zu einer Mischung von 40 g rauchender Schwefelsäure mit 25 %igem $SO_3$-Gehalt und 40 g Schwefelsäure-Monohydrat werden bei 2-5° C im Laufe von 10 Min. 5,0 g 1,4-Diketo-3-phenyl-6-(4' chlorphenyl)-pyrrolo-[3,4-c]-pyrrol, das gemäss dem Beispiel 6 der Europäischen Patentanmeldung Nr. 0184982 hergestellt wurde, eingetragen. Nach halbstündigem Rühren bei 5° C wird die Mischung während 17 Stunden bei 20° C weitergerührt. Die erhaltene Mischung wird auf Eiswasser ausgetragen. Das Gewicht der Mischung beträgt 420 g. Diese Mischung wird bei 70-75° C mit 30 g Natriumchlorid versetzt und 1 Stunde bei gleicher Temperatur weiter verrührt. Nach Abkühlen auf 20° C wird der ausgefallene Niederschlag abfiltriert, mit 5 %iger Natriumchloridlösung säurefrei gewaschen und bei 120° C getrocknet. Man erhält unter Berücksichtigung des Natriumchloridgehaltes 6,4 g des Natriumsalzes der Monosulfonsäure von 1,4-Diketo-3-phenyl-6-(4'-chlorphenyl)-pyrrolo-[3,4-c]-pyrrol der Formel

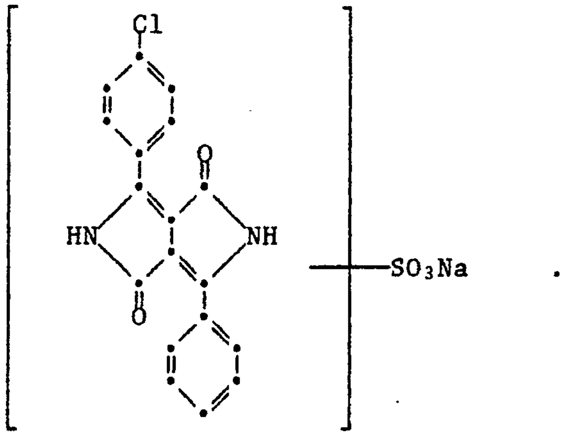

Dieses Produkt lässt sich analog Beispielen 12A/12B zu Pigmentgemischen verarbeiten, welche in Lacken zu einer Verbesserung der rheologischen Eigenschaften beiträgt.

Beispiel 18:

Zu einer Mischung von 40 g rauchender Schwefelsäure mit 25 %igem $SO_3$-Gehalt und 40 g Schwefelsäure-Monohydrat werden bei 2-5 °C, 5,0 g 1,4-Diketo-3-phenyl-6-(4'-methylphenyl)-pyrrolo-[3,4-c]-pyrrol, das analog dem Beispiel 6 der Europäischen Patentanmeldung Nr. 0184982 ausgehend von 4-Methylbenzonitril statt 4-Chlorbenzonitril hergestellt wurde, im Laufe von 10 Min. eingetragen. Die erhaltene Mischung wird darauf während 17 Stunden bei 20 °C verrührt und alsdann auf Eiswasser ausgetragen. Das Gewicht der Mischung beträgt 440 g. Die auf 70-75 °C erwärmte Mischung wird im Laufe von 30 Min. mit 40 g Natriumchlorid versetzt und eine Stunde bei 70-75 °C weiter verrührt. Der ausgefallene Niederschlag wird nach Abkühlung der Suspension auf 20 °C abfiltriert, mit 10 %iger Natriumchloridlösung säurefrei gewaschen und bei 120 °C getrocknet. Man erhält unter Berücksichtigung des Natriumchloridgehaltes 8,5 g des Dinatriumsalzes der Disulfonsäure von 1,4-Diketo-3-phenyl-6-(4'-methylphenyl)-pyrrolo-[3,4-c]-pyrrol der Formel

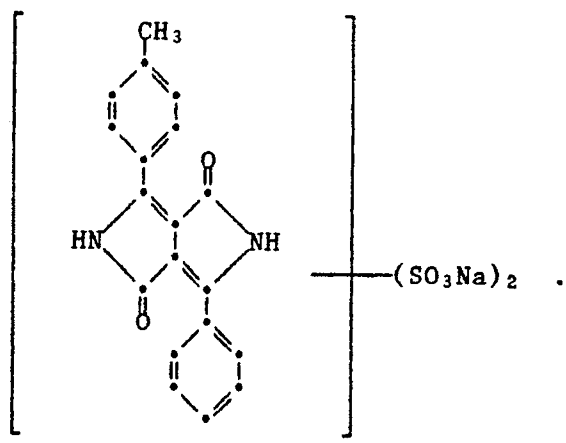

Dieses Produkt lässt sich analog Beispiel 12A/12B zu Pigmentgemischen verarbeiten, welche in Lacken zu einer Verbesserung der rheologischen Eigenschaften beiträgt.

Beispiel 19:

40 g rauchender Schwefelsäure von 25 %igem $SO_3$-Gehalt werden mit 40 g Schwefelsäure-Monohydrat

vermischt, und das erhaltene Gemisch wird auf 2-5° C gekühlt. Im Laufe von 10 Min. werden der Mischung unter Rühren 5,0 g 1,4-Diketo-3,6-di-(biphenyl)-pyrrolo-[3,4-c]-pyrrol, das analog dem Beispiel 38 der US-Patentschrift Nr. 4579949 ausgehend von p-Phenylbenzonitril statt Isophthalonitril hergestellt wird, zugefügt. Die erhaltene Mischung wird nach 18-stündigem Rühren bei 20° C auf Eiswasser ausgetragen. Das Gewicht der Mischung beträgt 470 g. Diese Mischung wird auf 70-75° C erhitzt und mit 20 g Natriumchlorid versetzt. Nach einer Stunde Rühren bei 70-75° C wird der Niederschlag bei 20° C abfiltriert, mit 4 %iger Natriumchloridlösung säurefrei gewaschen und bei 120° C getrocknet. Unter Berücksichtigung des Natriumchloridgehaltes erhält man 6,3 g des Dinatriumsalzes der 1,4-Diketo-3,6-di-(biphenyl)-pyrrolo-[3,4-c]-pyrrol-disulfonsäure der Formel

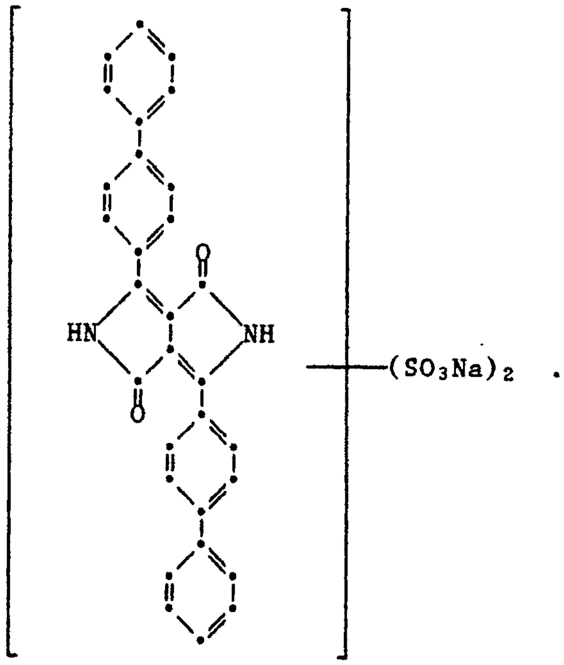

Dieses Produkt lässt sich analog Beispiel 12A/12B zu Pigmentgemischen verarbeiten, welche in Lacken zu einer Verbesserung der rheologischen Eigenschaften beiträgt.

Beispiel 20:

Ersetzt man in Beispiel 19 das 12,5 % $SO_3$ enthaltende Gemisch (aus rauchender Schwefelsäure und Schwefelsäure-Monohydrat) durch 80 g Schwefelsäure-Monohydrat und verfährt im übrigen nach den Angaben jenes Beispiels, so erhält man unter Berücksichtigung des Natriumchloridgehaltes 6,6 g des Dinatriumsalzes der 1,4-Diketo-3,6-di(biphenyl)-pyrrolo-[3,4-c]-pyrroldisulfonsäure obiger Formel.

## Patentansprüche

1. Stoffkompositionen enthaltend
   a) ein Pyrrolo-pyrrol der Formel I

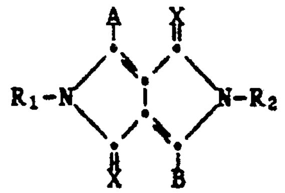

(I),

worin A und B gleiche oder voneinander verschiedene Alkyl- oder Aralkylreste, Aryl- oder heterocyclische aromatische Reste, $R_1$ und $R_2$ H-Atome, unsubstituierte oder substituierte Alkylreste, ferner Alkenyl-, Alkinyl-, Aralkyl-, Cycloalkyl-, Carbamoyl-, Alkylcarbamoyl-, Arylcarbamoyl-, Alkoxycarbonyl-, Aryl-, Alkanoyl- oder Aroylgruppen und X ein O- oder S-Atom bedeuten, und
b) ein Pyrrolo-pyrrol der oben angegebenen Formel I, das mindestens eine Gruppe der Formeln $-SO_3L$, $-CO_2L$, $-PO_3(L)_2$, $-N(R_4)(R_5)$,

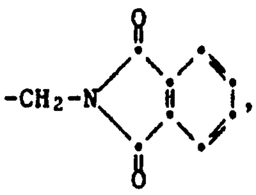 , 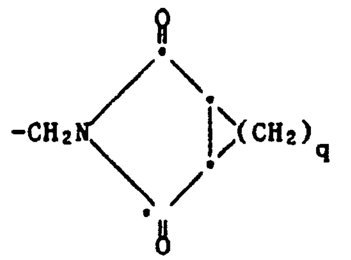 , 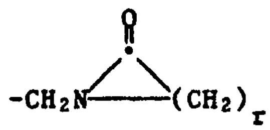 oder -NHCOR

aufweist, wobei L -H, eine Gruppe der Formeln

$$\frac{M^{+n}}{n}$$

oder $N^+H(R_3)(R_4)(R_5)$ darstellt, M ein ein-, zwei- oder dreiwertiges Metallkation, n die Zahlen 1, 2 oder 3, $R_3$, $R_4$ und $R_5$ unabhängig voneinander -H, Alkyl-, Aralkyl-, $C_5$-$C_6$-Cycloalkyl- oder Arylreste bedeuten oder $R_4$ und $R_5$ zusammen mit dem N-Atom einen 5- oder 6-gliedrigen heterocyclischen Rest bilden, q die Zahlen 0 bis 4, r die Zahlen 3 bis 5 und R Aryl oder $C_1$-$C_4$-Alkyl darstellen.

2. Stoffkompositionen gemäss Anspruch 1, worin A und B identisch sind und Aryl bedeuten.

3. Stoffkompositionen gemäss Anspruch 1, worin L -H oder eine Gruppe der Formel

$$\frac{M^{n+}}{n}$$

bedeutet, wobei M ein Alkali-, Erdalkali- oder Uebergangsmetallkation und n die Zahlen 1, 2 oder 3 bedeuten.

4. Stoffkompositionen gemäss Anspruch 1 enthaltend ein Pyrrolo-pyrrol der Formel II

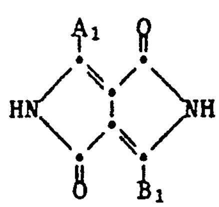 (II) ,

worin $A_1$ und $B_1$ unabhängig voneinander Arylreste darstellen, und
b) ein Pyrrolo-pyrrol der Formel III

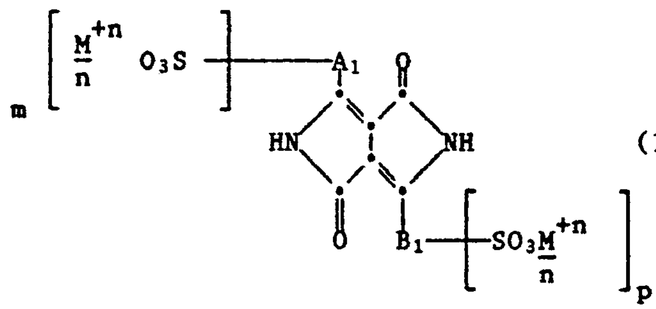

(III),

worin $A_1$ und $B_1$ die oben angegebene Bedeutung haben, M ein Alkali-, Erdalkali- oder Uebergangsmetallkation, n die Zahlen 1, 2 oder 3, und m und p unabhängig voneinander die Zahlen 0, 1, 2, 3 oder 4 bedeuten, wobei die Summe m und p mindestens 1 ist.

**5.** Stoffkompositionen gemäss Anspruch 4, worin $A_1$ und $B_1$ unsubstituiertes oder durch nichtwasserlöslichmachende Substituenten substituiertes Phenyl bedeuten.

**6.** Stoffkompositionen gemäss Anspruch 4, worin $A_1$ und $B_1$ Phenyl oder p-Chlorphenyl und m und p die Zahlen 0 oder 1 sind, wobei die Summe m und p mindestens 1 ist, n die Zahlen 1 oder 2 und M $Na^+$, $K^+$, $Mg^{2+}$, $Ca^{2+}$, $Sr^{2+}$ oder $Ba^{2+}$ bedeuten, wobei sich der Sulfonsäurerest auf dem Phenylkern in para-Stellung zum Diketopyrrolopyrrolgerüst befindet.

**7.** Stoffkompositionen gemäss Anspruch 1, worin das Mengenverhältniss der Komponente a) zur Komponente b) 99,9 bis 75 Gew.% zu 0,1 bis 25 Gew.% beträgt.

**8.** Verbindungen der Formel IV

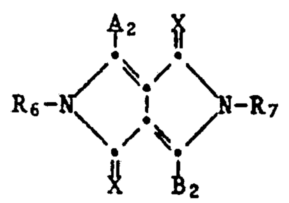

(IV),

enthaltend mindestens eine Gruppe der Formeln $-SO_3L$, $-CO_2L$, $-PO_3(L)_2$,

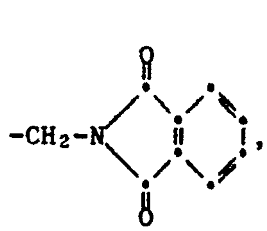 , 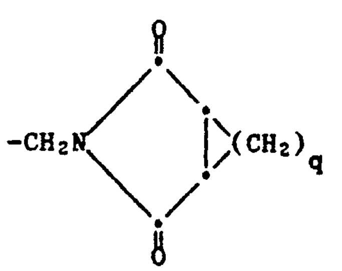 , 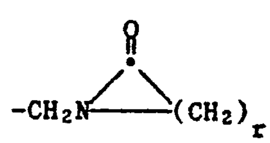

oder -NHCOR, worin $A_2$ und $B_2$ gleiche oder voneinander verschiedene Alkyl-, Aralkyl-, Aryl- oder heterocyclische aromatische Reste, $R_6$ und $R_7$ H-Atome, unsubstituierte oder substituierte Alkylreste, ferner Alkenyl-, Alkinyl-, Aralkyl-, Cycloalkyl-, Carbamoyl-, Alkylcarbamoyl-, Arylcarbamoyl-, Alkoxycarbonyl-, Aryl-, Alkanoyl-oder Aroylgruppen, und X ein O- oder S-Atom bedeuten, L -H oder eine Gruppe der Formeln

$$\frac{M^{+n}}{n}$$

oder $N^{+}H(R_8)(R_9)(R_{10})$ darstellt, M ein ein-, zwei- oder dreiwertiges Metallkation, n die Zahlen 1, 2 oder 3, $R_8$, $R_9$ und $R_{10}$ unabhängig voneinander -H, Alkyl-, Aralkyl-, $C_5$-$C_6$-Cycloalkyl- oder Arylreste bedeuten oder $R_9$ und $R_{10}$ zusammen mit dem N-Atom einen 5- oder 6-gliedrigen heterocyclischen Rest bilden, q die Zahlen 0 bis 4, r die Zahlen 3 bis 5 und R Aryl oder $C_1$-$C_4$-Alkyl darstellen, wobei L kein Natriumkation bedeuten darf, wenn die Verbindungen der Formel IV eine $-SO_3L$-Gruppe aufweist.

**9.** Verbindungen gemäss Anspruch 8, worin die Verbindungen der Formel IV die Formel V

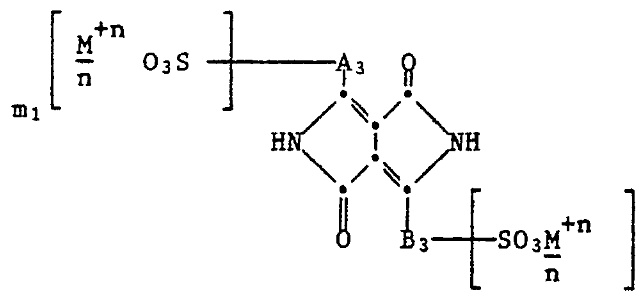

(V) aufweisen,

worin $A_3$ und $B_3$ Phenyl oder Naphthyl und $m_1$ und $p_1$ die Zahlen 0 oder 1 bedeuten, wobei die Summe $m_1$ und $p_1$ mindestens 1 ist, ferner n die Zahlen 1, 2 oder 3 und M ein Erdalkali- oder Uebergangsmetallkation, $Li^+$ oder $K^+$ bedeuten.

**10.** Verbindungen der Formel V gemäss Anspruch 9, worin $A_3$ und $B_3$ Phenyl, und $m_1$ und $p_1$ die Zahlen 0 oder 1 bedeuten, wobei die Summe $m_1$ und $p_1$ mindestens 1 ist, n die Zahlen 1 oder 2 und M $K^+$, $Ca^{2+}$, $Mg^{2+}$, $Sr^{2+}$, $Ba^{2+}$, $Zn^{2+}$oder $Cd^{2+}$ bedeuten.

**11.** Verfahren zum Färben von hochmolekularem organischem Material, dadurch gekennzeichnet, dass eine Stoffkomposition gemäss Anspruch 1 verwendet wird.

**12.** Verfahren zum Färben von hochmolekularem organischem Material, dadurch gekennzeichnet, dass eine Verbindung gemäss Anspruch 8 verwendet wird.

**13.** Hochmolekulares organisches Material enthaltend eine Stoffkomposition gemäss Anspruch 1.

**14.** Hochmolekulares organisches Material enthaltend eine Verbindung gemäss Anspruch 8.

## Claims

**1.** A composition comprising
a) a pyrrolopyrrole of formula I

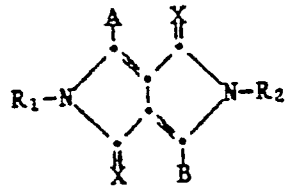

(I),

in which A and B are identical or mutually different alkyl, aralkyl, aryl or heterocyclic aromatic

radicals, $R_1$ and $R_2$ are atoms, unsubstituted or substituted alkyl radicals, and also alkenyl, alkynyl, aralkyl, cycloalkyl, carbamoyl, alkylcarbamoyl, arylcarbamoyl, alkoxycarbonyl, aryl, alkanoyl or aroyl groups, and X is an O or S atom, and
b) a pyrrolopyrrole of the above formula I which contains at least one group of the formulae $-SO_3L$, $-CO_2L$, $-PO_3(L)_2$, $-N(R_4)(R_5)$,

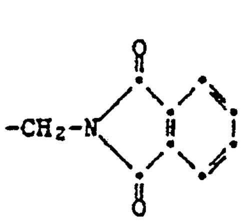,
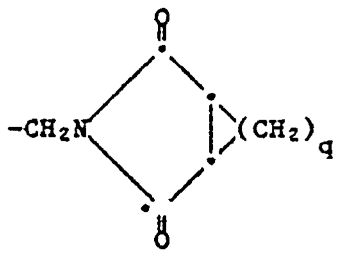,
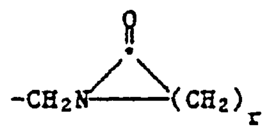 or -NHCOR

in which L is -H, a group of formula

$$\frac{M^{+n}}{n}$$

or $N^{+}H(R_3)(R_4)(R_5)$, M is a monovalent, divalent or trivalent metal cation, n is the number 1, 2 or 3, $R_3$, $R_4$ and $R_5$ are each independently of the others -H, alkyl, aralkyl, $C_5$-$C_6$cycloalkyl or aryl radicals, or $R_4$ and $R_5$, together with the N atom, form a 5- or 6-membered heterocyclic radical, q is a number from 0 to 4, r is a number from 3 to 5 and R is aryl or $C_1$-$C_4$alkyl.

2. A composition according to claim 1, wherein A and B are identical and are aryl.

3. A composition according to claim 1, wherein L is -H or a group of formula

$$\frac{M^{n+}}{n},$$

in which M is an alkali metal cation,
an alkaline earth metal cation or a transition metal cation, and n is the number 1, 2 or 3.

4. A composition according to claim 1, comprising a pyrrolopyrrole of formula II

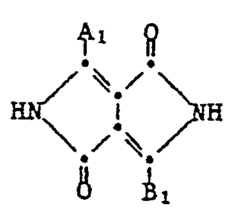 (II) ,

in which $A_1$ and $B_1$ are each independently of the other aryl radicals, and
b) a pyrrolopyrrole of formula III

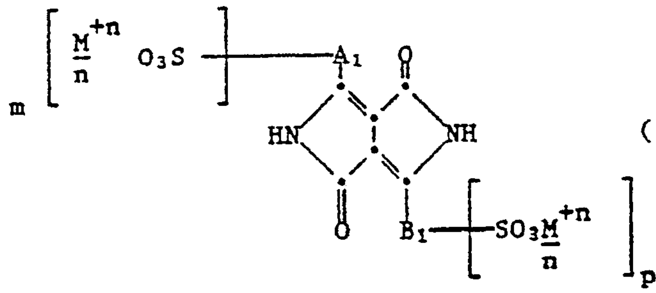

(III),

in which $A_1$ and $B_1$ are as defined above, M is an alkali metal cation, an alkaline earth metal cation or a transition metal cation, n is the number 1, 2 or 3, and m and p are each independently of the other 0, 1, 2, 3 or 4, with the sum of m and p being at least 1.

5. A composition according to claim 4, wherein $A_1$ and $B_1$ are phenyl which is unsubstituted or substituted by non-water-solubilising substituents.

6. A composition according to claim 4, wherein $A_1$ and $B_1$ are phenyl or p-chlorophenyl and m and p are the number 0 or 1, with the sum of m and p being at least 1, n is the numbers 1 or 2 and M is $Na^+$, $K^+$, $Mg^{2+}$, $Ca^{2+}$, $Sr^{2+}$ or $Ba^{2+}$, and the sulfonic acid radical on the phenyl ring is in the para-position to the diketopyrrolopyrrole structure.

7. A composition according to claim 1, wherein the quantity ratio of component a) to component b) is 99.9 to 75 % by weight to 0.1 to 25 % by weight.

8. A compound of formula IV

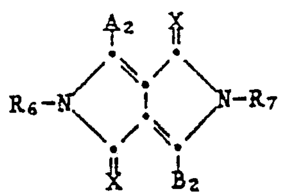

(IV),

containing at least one group of the formulae $-SO_3L$, $-CO_2L$, $-PO_3(L)_2$,

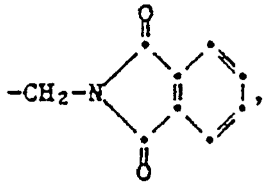

,

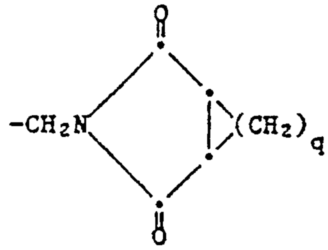

,

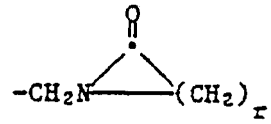

or -NHCOR, in which $A_2$ and $B_2$ are identical or mutually different alkyl, aralkyl, aryl or heterocyclic aromatic radicals, $R_6$ and $R_7$ are H atoms, unsubstituted or substituted alkyl radicals, and also alkenyl, alkynyl, aralkyl, cycloalkyl, carbamoyl, alkylcarbamoyl, arylcarbamoyl, alkoxycarbonyl, aryl, alkanoyl, or aroyl groups, and X is an O or S atom, L is -H or a group of formula

$$\frac{M^{+n}}{n}$$

or $N^+H(R_8)(R_8)(R_{10})$, M is a monovalent, divalent or trivalent metal cation, n is the numbers 1, 2 or 3,

$R_8$, and $R_{10}$ are each independently of the others -H, alkyl, aralkyl, $C_5$-$C_6$cycloalkyl or aryl radicals, or $R_9$ and $R_{10}$, together with the N atom form a 5- or 6-membered heterocyclic radical, q is a number from 0 to 4, r is a number from 3 to 5 and R is aryl or $C_1$-$C_4$alkyl, where L may not be a sodium cation if the compounds of formula IV contain an $-SO_3L$ group.

**9.** A compound according to claim 8, wherein the compound of formula IV has the formula V

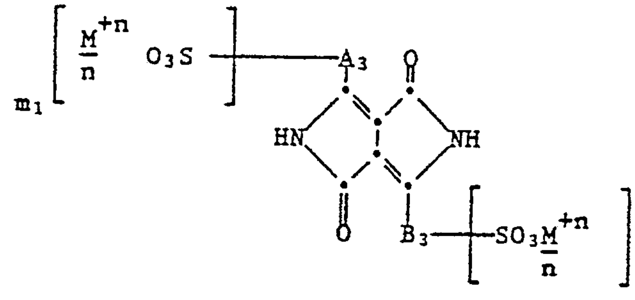

(V)

in which $A_3$ and $B_3$ are phenyl or naphthyl and $m_1$ and $p_1$ are the numbers 0 or 1, with the sum of $m_1$ and $p_1$ being at least 1, and n is the number 1, 2 or 3, and M is an alkaline earth metal cation or a transition metal cation, $Li^+$ or $K^+$.

**10.** A compound of formula V according to claim 9, in which $A_3$ and $B_3$ are phenyl and $m_1$ and $p_1$ are the number 0 or 1, with the sum of $m_1$ and $p_1$ being at least 1, n is the number 1 or 2, and M is $K^+$, $Ca^{2+}$, $Mg^{2+}$, $Sr^{2+}$, $Ba^{2+}$, $Zn^{2+}$ or $Cd^{2+}$.

**11.** A process for colouring high molecular weight organic material, which comprises using a composition as claimed in claim 1.

**12.** A process for colouring high molecular weight organic material, which comprises using a compound as claimed in claim 8.

**13.** High molecular weight organic material containing a composition as claimed in claim 1.

**14.** High molecular weight organic material containing a compound as claimed in claim 8.

**Revendications**

**1.** Compositions qui contiennent :

a) un pyrrolopyrrole de formule I ci-dessous

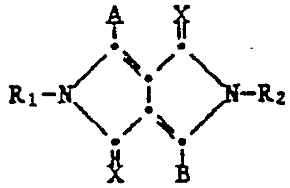

(I),

dans laquelle A et B qui peuvent être identiques ou différents l'un de l'autre sont des alkyles, des aralkyles, des aryles ou des radicaux aromatiques hétérocycliques, $R_1$ et $R_2$ des atomes d'hydrogène, des alkyles avec ou sans substituants ou encore des groupes alcényles, alcynyles, aralkyles, cycloalkyles, carbamoyles, alkylcarbamoyles, arylcarbamoyles, alcoxycarbonyles, aryles, alcanoyles ou aroyles, et X désigne un atome d'oxygène ou de soufre, avec

b) un pyrrolo-pyrrole de formule I ci-dessus mais avec au moins un groupe de formule $-SO_3L$, $-CO_2L$, $-PO_3(L)_2$, $-N(R_4)(R_5)$,

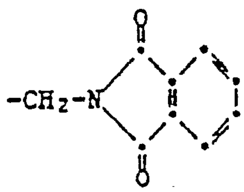, 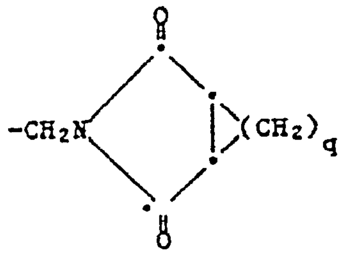, 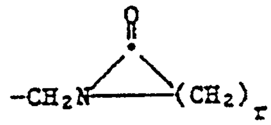 ou -NHCOR

L désignant -H ou un groupe $M^{+n}$ ou $N^{+}H(R_3)(R_4)$ $(R_5)$, M étant un cation de métal monovalent, bivalent ou trivalent et n le nombre 1, 2 ou 3, $R_3$, $R_4$ et $R_5$ désignant, indépendamment les uns des autres des atomes d'hydrogène ou bien des alkyles, des aralkyles, des cycolalkyles en $C_5$-$C_6$ ou des aryles, ou bien $R_4$ et $R_5$ forment ensemble et avec l'atome d'azote un radical hétérocyclique à cinq ou six chaînons, q étant un nombre de 0 à 4, r un nombre de 3 à 5 et R un aryle ou un alkyle en $C_1$-$C_4$.

**2.** Compositions selon la revendication 1 dans lesquelles A et B sont identiques et sont des aryles.

**3.** Compositions selon la revendication 1 dans lesquelles L désigne l'hydrogène ou un groupe

$$\frac{M^{n+}}{n},$$

M étant un cation de métal acalin ou alcalino-terreux ou d'un métal de transition et n le nombre 1, 2 ou 3.

**4.** Compositions selon la revendication 1 qui contiennent un pyrrolo-pyrrole de formule II

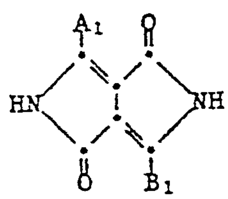 (II) ,

dans laquelle $A^1$ et $B_1$ sont des aryles indépendants l'un de l'autre, et

b) un pyrrolo-pyrrole de formule III

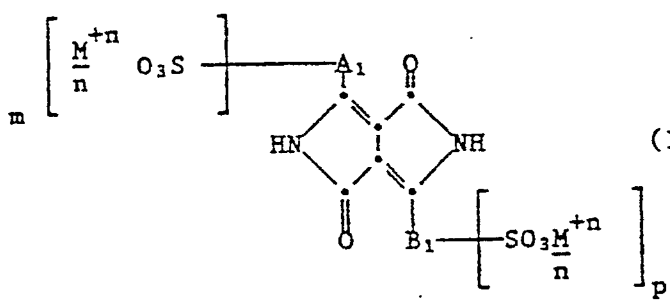 (III),

dans laquelle $A_1$ et $B_1$ ont les significations ci-dessus, M désigne un cation de métal alcalin ou alcalino-terreux ou d'un métal de transition, n est le nombre 1, 2 ou 3 et m et p sont chacun, indépendamment l'un de l'autre, le nombre 0, 1, 2, 3 ou 4, la somme m + p étant au moins égale à 1.

**5.** Compositions selon la revendication 4 dans lesquelles $A_1$ et $B_1$ sont des phényles sans substituants ou avec des substituants non-hydrosolubilisants.

**6.** Compositions selon la revendication 4 dans lesquelles $A_1$ et $B_1$ sont le groupe phényle ou p-chlorophényle et m et p le nombre 0 ou 1, la somme m + p étant au moins égale à 1, n est le nombre 1 ou 2 et M le cation $Na^+$, $K^+$, $Mg^{2+}$, $Ca^{2+}$, $Sr^{2+}$ ou $Ba^{2+}$, le radical d'acide sulfonique du cycle phénylique étant à la position para par rapport au système dicétopyrrolo-pyrrolique.

**7.** Compositions selon la revendication 1, dont les proportions mutuelles des composants a) et b) sont respectivement de 99,9 à 75 % en poids pour 0,1 à 25 %.

**8.** Les composés de formule IV

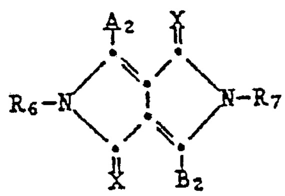

(IV),

comprenant un ou plusieurs des groupes $-SO_3L$, $-CO_2L$, $-PO_3(L)_2$,

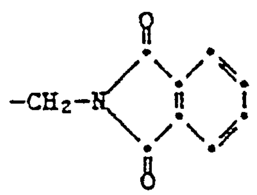, 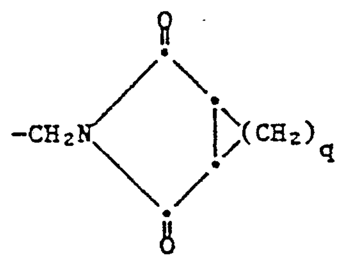, 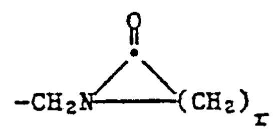

et -NHCOR,

formule dans laquelle $A_2$ et $B_2$, qui peuvent être identiques ou différents l'un de l'autre, sont des alkyles, des aralkyles, des aryles ou des radicaux aromatiques hétérocycliques, $R_6$ et $R_7$ des atomes d'hydrogène, des alkyles avec ou sans substituants ou encore des alcényles, alcynyles, aralkyles, cycloalkyles, benzyles, carbamoyles, alkylcarbamoyles, arylcarbamoyles, alcoxycarbonyles, aryles, alcanoyles ou aroyles, et X désigne un atome d'oxygène ou de soufre, L réprésentant -H ou un groupe

$$\frac{M^{+n}}{n}$$

ou $N^+H(R_8)(R_9)(R_{10})$, M un cation de métal monovalent, divalent ou trivalent, n le nombre 1, 2 ou 3 et $R_8$, $R_9$ et $R_{10}$ étant chacun, indépendamment les uns des autres, l'hydrogène ou bien un alkyle, un aralkyle, un cycloalkyle en $C_5$-$C_6$ ou un aryle, ou bien $R_9$ et $R_{10}$ formant ensemble et avec l'atome d'azote un radical hétérocyclique à cinq ou six chaînons, q est un nombre de 0 à 4, r un nombre de 3 à 5 et R un aryle ou un alkyle en $C_1$-$C_4$, mais L n'étant pas le cation sodium si le composé de formule IV comporte un groupe $-SO_3L$.

**9.** Composés selon la revendication 8, de formule V ci-dessous :

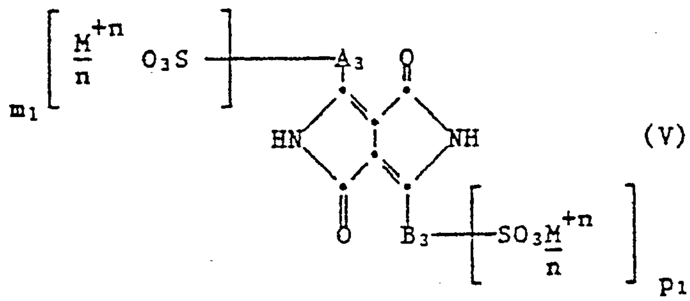

(V)

dans laquelle $A_3$ et $B_3$ sont le groupe phényle ou naphtyle et $m_1$ et $P_1$ le nombre 0 ou 1, la somme $m_1 + P_1$ étant au moins égale à 1, n est le nombre 1, 2 ou 3 et M le cation d'un métal alcalino-terreux ou de transition, $Li^+$ ou $K^+$.

**10.** Composés de formule V selon la revendication 9, dans lesquels $A_3$ et $B_3$ sont le groupe phényle et $m_1$ et $p_1$ le nombre 0 ou 1, la somme $m_1 + P_1$ étant au moins égale à 1, n est le nombre 1 ou 2 et M le cation $K^+$, $Ca^{2+}$, $Mg^{2+}$, $Sr^{2+}$, $Ba^{2+}$, $Zn^{2+}$ ou $Cd^{2+}$.

**11.** Procédé de coloration, teinture ou pigmentation, de matières organiques macro-moléculaires, procédé caractérisé en ce que l'on utilise une composition selon la revendication 1.

**12.** Procédé de coloration de matières organiques macro-moléculaires, caractérisé en ce que l'on utilise un composé selon la revendication 8.

**13.** Matières organiques macro-moléculaires qui contiennent une composition selon la revendication 1.

**14.** Matières organiques macro-moléculaires qui contiennent un composé selon la revendication 8.